# EUROPEAN PATENT SPECIFICATION

(11) **EP 3 840 632 B1**
(45) Date of publication and mention of the grant of the patent: **05.03.2025**
(21) Application number: 19773207.6
(22) Date of filing: 19.08.2019
(51) Int. Cl.: A61B 5/00, A61B 5/06, A61B 5/20, A61B 17/29, A61B 17/00, A61B 90/00

(54) **A SYSTEM WITH APPLIED STIMULATION USED TO DETECT OR DIFFERENTIATE TISSUE OR ARTIFACT**
SYSTEM MIT ANGEWANDTER STIMULATION ZUR ERKENNUNG ODER DIFFERENZIERUNG VON GEWEBE ODER ARTEFAKTEN
SYSTÈME AVEC STIMULATION APPLIQUÉE UTILISÉE POUR DÉTECTER OU DIFFÉRENCIER UN TISSU OU UN ARTEFACT

(30) Priority: 20.08.2018 US 201862719792 P; 31.12.2018 US 201862786666 P
(43) Date of publication of application: 30.06.2021
(73) Proprietor: Briteseed, LLC, Chicago, IL 60625 (US)
(72) Inventor: LE ROLLAND, Paul, Campbell, CA 95008 (US); CHATURVEDI, Amal, San Jose, CA 95134 (US)
(74) Representative: Kraus & Lederer PartGmbB
(86) International application number: PCT/US2019/047076
(87) International publication number: WO 2020/041203

(56) References cited:
- WO-A1-2016/134327
- US-A1- 2015 066 000
- US-A1- 2017 042 445

## Description

### Background

This patent is directed to a system and method used to detect or differentiate tissue or an artifact, such as a vessel, and in particular to a system and method used to detect or differentiate tissue or an artifact where stimulation is applied to the tissue or artifact. US 2017/042445 A1 is directed at a method for identifying a ureter during a medical procedure, the method comprising: providing an electrical stimulator comprising: a shaft having at least one electrode; and a power supply connected to the at least one electrode for providing an electrical signal to the at least one electrode; advancing the shaft so that the at least one electrode contacts tissue; operating the power supply so that the electrical signal is applied to the tissue; and visually observing the tissue or listening to an audio signal obtained by converting an EMG signal, to determine the presence of a ureter in the tissue. US 2015/066000 A1 discloses an instrumented surgical device comprising: a surgical tool to perform a surgical procedure within a surgical field; and an optical sensor operatively connected to the surgical tool, wherein the sensor monitors the surgical field for a structural artefact. WO 2016/134327 A1 discloses a surgical system used to determine the presence or absence of a vessel and determine a size of the vessel within a region proximate to a working end of a surgical instrument, comprising: at least one light emitter disposed at the working end of the surgical instrument; at least one light sensor disposed at the working end of the surgical instrument opposite the at least one light emitter, the at least one light sensor adapted to generate a signal comprising a first pulsatile component and a second non-pulsatile component; and a controller coupled to the at least one light sensor, the controller comprising a splitter to separate the first pulsatile component from the second non-pulsatile component and an analyzer to quantify the size of the vessel within the region proximate to the working end of the surgical instrument based on the first pulsatile component.

Systems and methods that detect or differentiate tissue(s) or artifacts, such as vessels, in the surgical field during a surgical procedure provide valuable information to the surgeon or surgical team. U.S. hospitals lose billions of dollars annually in unreimbursable costs because of inadvertent vascular damage during surgery. In addition, the involved patients face a mortality rate of up to 32%, and likely will require corrective procedures and remain in the hospital for up to an additional nine days, resulting in tens, if not hundreds, of thousands of dollars in added costs of care. Consequently, there is significant value to be obtained from methods and systems that permit accurate detection or differentiation of tissue(s) or artifacts, such as vessels, in the surgical field, such that these costs may be reduced or avoided.

Systems and methods that provide such information are particularly important during minimally invasive surgical procedures. Traditionally, surgeons have relied upon tactile sensation during surgical procedures both to identify blood vessels, for example, and to avoid inadvertent damage to these vessels. Because of the shift towards minimally invasive procedures, including laparoscopic and robotic surgeries, surgeons have lost the ability to use direct visualization and the sense of touch to make determinations as to the presence of tissue(s) or artifacts in the surgical field, and to differentiate between those tissue(s) or artifacts. Consequently, surgeons must make the determination whether certain tissues or artifacts are present in the surgical field based primarily on convention and experience. Unfortunately, anatomical irregularities frequently occur because of congenital anomalies, scarring from prior surgeries, and body habitus (e.g., obesity). Systems and methods that would permit surgeons to determine the presence and/or the characteristics of tissue(s) and artifacts in the surgical field during surgery (potentially in real time or near real time) under such conditions would provide a significant advantage.

On the other hand, while it would be advantageous to include systems and methods that provide such information about the surgical field, the adoption of these systems and methods would be impeded if the systems and methods made the surgical procedure more complicated. Consequently, it is advantageous that the systems and methods do not involve significant thought on the part of the surgeon or surgical team in using the system or method for detecting or differentiating tissue or an artifact, like a vessel, or significant preparation of the surgical field or the patient for use of the system or method.

As set forth in more detail below, the present disclosure describes a systems and methods embodying advantageous alternatives to the existing systems and methods, which may provide for improved detection and/or differentiation for avoidance or isolation of tissue or artifacts, such as vessels, without undue complication of the surgical instrument or surgical procedure.

### Summary

The invention is defined by the appended claims and relates to a surgical system. Methods are useful to understand the claimed invention.
According to an aspect of the present disclosure, a surgical system includes a stimulation generator, at least one sensor, and a controller coupled to the at least one sensor. The controller is configured to determine if a tissue or an artifact proximate to the at least one sensor has undergone a change in response to a stimulation applied by the stimulation generator, and to indicate if the tissue or artifact is present within a region proximate to a working end of a surgical instrument based on the presence or absence of the change in response to a stimulation applied by the stimulation generator.

According to another aspect of the present disclosure, a method for operating a surgical system to determine whether tissue or an artifact is in the surgical field is provided. The method includes obtaining a first set of sensor data from at least one sensor, applying a stimulation to the surgical field after obtaining the first set of sensor data, obtaining a second set of sensor data from the at least one sensor after applying the stimulation to the surgical field, and comparing the first set of sensor data with the second set of sensor data to determine whether tissue or an artifact is in the surgical field.

According to an aspect of the present disclosure, a surgical system includes a stimulation generator, at least one sensor disposed at a working end of a surgical instrument, and a controller coupled to the at least one sensor. The controller is configured to obtain data from the at least one sensor over time in response to a stimulation applied by the stimulation generator, analyze the data obtained from the at least one sensor over time to generate sensor data analysis results, apply a signal processing method and/or a pattern matching to the sensor data analysis results, and indicate if a tissue or artifact is present within a region proximate to the working end of the surgical instrument based on the signal processing method or pattern matching.

According to another aspect of the present disclosure, a method is provided for operating a surgical system to determine whether tissue or an artifact is in the surgical field. The method includes applying a stimulation to the surgical field, obtaining data from at least one sensor over time, the at least one sensor disposed at a working end of a surgical instrument, analyzing the data obtained from the at least one sensor over time to generate sensor data analysis results, applying a signal processing method and/or a pattern matching to the sensor data analysis results, and indicating if the tissue or artifact is present within a region proximate to a working end of a surgical instrument based on the signal processing method or pattern matching.

### Brief Description of the Drawings

The disclosure will be more fully understood from the following description taken in conjunction with the accompanying drawings. Some of the figures may have been simplified by the omission of selected elements for the purpose of more clearly showing other elements. Such omissions of elements in some figures are not necessarily indicative of the presence or absence of particular elements in any of the exemplary embodiments, except as may be explicitly delineated in the corresponding written description. None of the drawings is necessarily to scale.
Fig. 1 is a schematic diagram of a surgical system according to an embodiment of the present disclosure;
Fig. 2 is a circuit diagram of an electrical stimulation generator that may be used in the surgical system of Fig. 1;
Fig. 3 is an enlarged, fragmentary view of a transmittance-based embodiment of a sensor that may be used in the surgical system of Fig. 1 with light emitters and light sensors, and a section of a vessel illustrated as disposed between the light emitters and light sensors;
Fig. 4 is an enlarged, side view of the jaws of a surgical instrument that may be used in the surgical system of Fig. 1, illustrating the configuration of the electrodes coupled to the electrical stimulation generator;
Fig. 5 is an enlarged, fragmentary view of an embodiment of the surgical instrument of Fig. 1 fitted with a reflectance-based system with light emitter and light sensor with fixed spacing, and a section of an vessel illustrated as proximate the light emitter and light sensors;
Fig. 6 is an enlarged, fragmentary view of another embodiment of a surgical instrument fitted with a reflectance-based system with light emitter and light sensor having an adjustable spacing relative to each other, and a section of a vessel illustrated as proximate the light emitter and light sensor;
Fig. 7 is a flowchart of a method used by any of the foregoing systems to detect or differentiate one or more regions of interest within the signal(s) received from an array of light sensors, which detection or differentiation of one or more regions of interest leads to detection or differentiation of tissue or one or more artifacts;
Fig. 8 is a flowchart of an analysis method used by any of the foregoing systems to detect or differentiate one or more regions of interest within the signal(s) received from an array of light sensors, which detection or differentiation of one or more regions of interest leads to detection or differentiation of tissue or one or more artifacts;
Fig. 9 is a schematic diagram of a surgical system according to an embodiment of the present disclosure, in combination with an embodiment of a video system, illustrating the surgical system in use with the video system; and
Fig. 10 is a schematic diagram of a surgical system according to an embodiment of the present disclosure, in combination with another embodiment of a video system, illustrating the surgical system in use with the video system.

### Detailed Description of Various Embodiments

The embodiments described herein provide systems and methods for detecting or differentiating tissues and/or artifacts, such as vessels. Such a system may include or a method may use a stimulation generator and at least one sensor, the at least one sensor coupled to a controller that is configured to determine if a tissue or an artifact proximate to the at least one sensor has undergone a change in response to a stimulation applied by the stimulation generator, and to indicate if the tissue or artifact is present within a region proximate to a working end of a surgical instrument based on the presence or absence of the change in response to a stimulation applied by the stimulation generator.

In the context of the claimed invention, the stimulation generator is an electrical stimulation generator. Further, the electrical stimulation generator may apply an electrical field or current to the tissue or artifact, which field or current may be varied to change the characteristics of the response of the tissue or artifact. The electrical stimulation generator may include or be electronically connected or coupled to, for example, one or more surface electrodes, one or more inserted electrodes, or one or more intraoperative probes or needles. Alternatively, in further configurations useful to understand the claimed invention, the stimulation generator may apply pressure or heat to the tissue or artifact to obtain a change in the tissue or artifact. In embodiments useful for detecting and differentiating a ureter, the stimulation generator is an electrical stimulation generator.

For some embodiments, and in the context of the claimed invention, the at least one sensor includes at least one light emitter (or simply the light emitter) and at least one light sensor or detector (or simply the light sensor). The light emitter and the light sensor may be disposed at the working end of the surgical instrument. The light emitter and light sensor may operate according to a transmittance-based approach, such that the light sensor is disposed opposite and facing the light emitter, for example on opposite jaws of a surgical instrument. The light emitter and light sensor may instead operate according to a reflectance-based approach, such that the light emitter and the light sensor may face in a common direction and with fixed or variable spacing therebetween.

For other embodiments, embodiments useful to understand the claimed invention, the at least one sensor may include a measurement of the impedance between electrodes of the stimulation generator before and after stimulation. Further embodiments for the at least one sensor may include measurements of the change in the electrical potential of the tissue or changes in the resistance between the electrodes of the stimulation generator before and after stimulation.

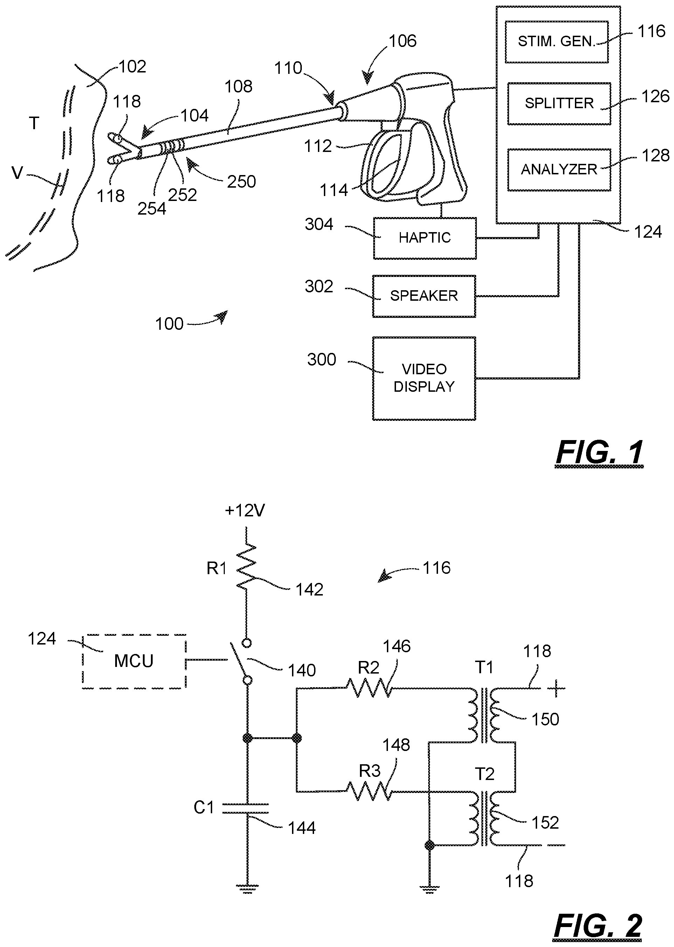

Turning first then to Fig. 1, an embodiment of a surgical system 100 is illustrated, which system 100 may be used to determine, for example, a characteristic (e.g., presence, diameter, etc.) of a vessel, V, within a region 102 of tissue, T, proximate to a working end 104 of a surgical instrument 106. It will be understood that the vessel V may be connected to other vessels with the region 102 of tissue T, and in addition, the vessel V may extend beyond the region 102 so as to be in fluid communication with other organs (e.g., the heart) also found in the body of the patient. Furthermore, while the tissue T appears in Fig. 1 to surround fully the vessel V (in terms of both circumference and length) to a particular depth, this need not be the case in all instances where the system 100 is used. For example, the tissue T may only partially surround the circumference of and/or only surround a section of the length of the vessel V, or the tissue T may overlie the vessel V in a very thin layer. As further non-limiting examples, the vessel V may be a ureter, and the tissue T may be connective tissue, adipose tissue and/or liver tissue.

According to the illustrated embodiments, the working end 104 of the surgical instrument 106 is also a distal end of a shaft 108. Consequently, the working end and the distal end will be referred to as working end 104 or distal end 104. The shaft 108 also has a proximal end 110, and a grip or handle 112 (referred to herein interchangeably as grip 112) is disposed at the proximal end 110 of the shaft 108. The grip 112 is designed in accordance with the nature of the instrument 106; as to the thermal ligation device illustrated in Fig. 1, the grip 112 may be a pistol-type grip including a trigger 114. As one alternative, finger rings arranged in a generally scissors-type grip may be used.

While the working or distal end 104 and the proximal end 110 with grip 112 are illustrated as disposed at opposite-most ends of the shaft 108, it will be recognized that certain surgical instruments have working ends (where a tool tip is attached, for example) disposed on the opposite-most ends of the shaft and a gripping region disposed intermediate to the opposite working ends. In accordance with the terms "distal" and "proximal" as used herein, the working ends of such an instrument are referred to herein as the distal ends and the gripping region as the proximal end. Relative to the illustrated embodiments, however, the distal and proximal ends are located at opposite-most (or simply opposite) ends of the shaft 108.

As mentioned above, a stimulation generator 116 is provided, which stimulation generator 116 may be connected or coupled to one or more electrodes 118, which electrodes 118 may be disposed at the working end 104 of the surgical instrument 106. As illustrated, there are two electrodes 118 provided at the working end 104 of the surgical instrument 106. These electrodes 118 may be coupled to the stimulation generator 116 via one or more wires or leads that travel along the shaft 108 of the surgical instrument 106. While the stimulation generator 116 is illustrated as separate from the surgical instrument 106 in Fig. 1, according to other embodiments, the stimulation generator 116 may be disposed on or in the surgical instrument 106.

In operation, the stimulation generator 116 provides an electrical current, for example a pulsed electrical current, to the working end 104 of the surgical instrument 106, and in particular the electrodes 118. When the electrodes 118 are disposed on or near certain muscle tissue, for example, the application of the electrical current, in particular pulsed electrical current, causes the muscle tissue to respond (e.g., contract). Specifically, it is known that when a patient is under general anesthetic for intra-abdominal operation, the skeletal muscle tissue of the patient will not respond to electrical stimulation, while the smooth muscle will respond. As such, an electrical stimulation applied to an artifact, such as a ureter, will cause the ureteral muscles to contract up and down the length of the ureter, resulting in movement of the ureter, which movement may be sensed even if other tissue (e.g., connective or adipose tissue) limits or prevents direct visualization of the ureter.

While such movement may be detectable through direct visualization or by remote visualization (e.g., on a video monitor such as commonly used in laparoscopic or robotic procedures), visualization of any particular point within the surgical field can be challenging at the best of times. Tissues and fluids can obscure the field, such that movement may be challenging to detect simply by looking for visual cues. Consequently, enhanced and/or automated sensing techniques can be of assistance when using stimulation of the ureter to aid in its detection and/or differentiation.

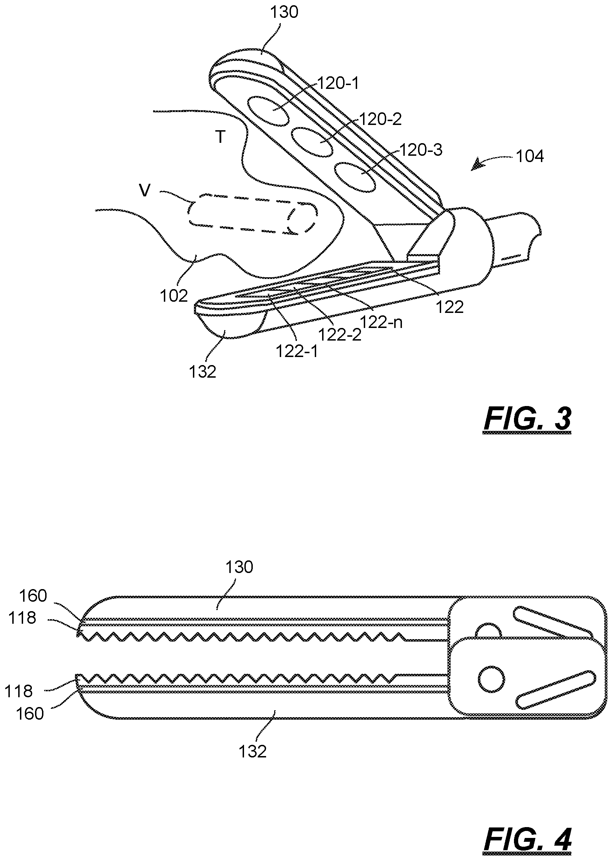

According to the claimed invention and as illustrated in Figs. 3-6, the surgical system 100 includes at least one sensor having at least one light emitter 120 (or simply the light emitter 120) and at least one light sensor or detector 122 (or simply the light sensor 122). See Figs. 3, 5, 6. As illustrated, the light emitter 120 is disposed at the working end 104 of the surgical instrument 106, and the light sensor 122 is also disposed at the working end 104 of the surgical instrument 106. A controller 124 is coupled to the light emitter 120 and the light sensor 122, which controller 124 may include a splitter 126 and an analyzer 128 as explained below. See Fig. 1.

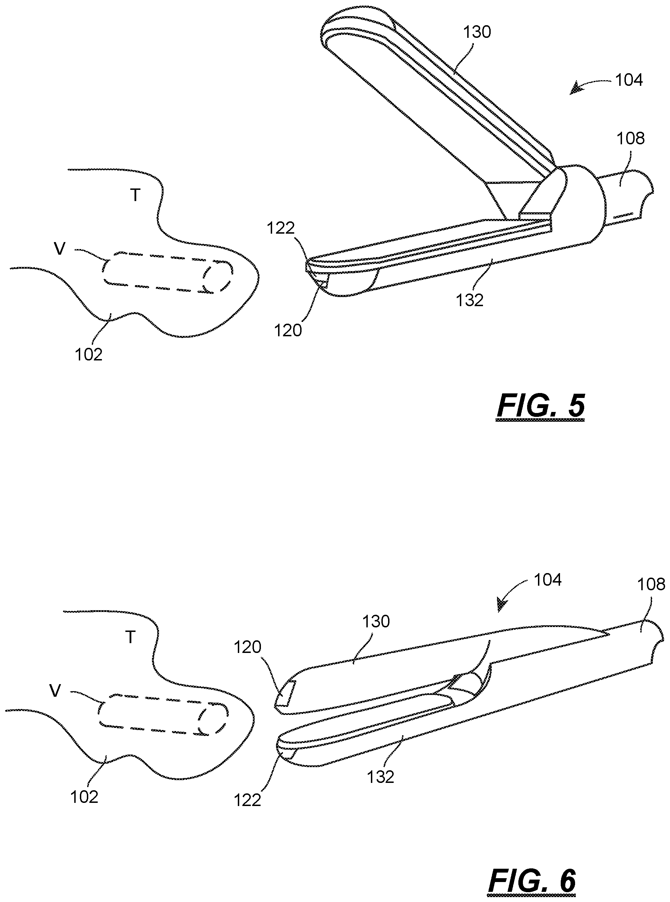

The at least one sensor may operate according to a transmittance-based approach, such that the light sensor(s) 122 is/are disposed opposite and facing the light emitter(s) 120, for example on opposite jaws 130, 132 of a surgical instrument 106 as illustrated in Fig. 3. In addition or the alternative, the system 100 may include a reflectance-based system (for forward-facing vessel and/or tissue detection), such that the light emitter 120 and the light sensor 122 may face in a common direction (at the tip, as illustrated, or to the side of the jaws 130, 132, for example) and with fixed spacing therebetween, for example on a single jaw 132 of a two-jaw device (see Fig. 5). The light emitter 120 and the light sensor 122 of a reflectance-based system instead may be constructed such that the spacing between the light emitter 120 and the light sensor 122 may be adjusted, for example by positioning the light emitter 120 at the end or tip (or side) of one of the jaws 130 of a two-jaw device and the light sensor 122 at the end or tip (or side) of the other the jaws 132 of the two-jaw device (see Fig. 6). As to the operation of such reflectance-based systems, see PCT Pub. No. WO 2017/139624.

It will be recognized that while embodiments have been illustrated wherein the surgical instrument 106 has jaws 130, 132, the system could be used with surgical instruments or tools that do not have jaws, or that are blunt-tipped. It will be further recognized that the electrode and sensor placement may be similar to the embodiments wherein the sensor is placed on a single jaw, e.g., on the end of the blunt-tipped instrument or tool or to the side of the blunt-tipped instrument or tool.

The light emitter 120 may be configured to emit light of at least one wavelength. For example, the light emitter 120 may emit light having a wavelength of 660 nm. This may be achieved with a single element, or a plurality of elements (which elements may be arranged or configured into an array, for example, as explained in detail below). In a similar fashion, the light sensor 122 is configured to detect light at the at least one wavelength (e.g., 660 nm). According to the embodiments described herein, the light sensor 122 includes a plurality of elements, which elements are arranged or configured into an array.

According to certain embodiments, the light emitter 120 may be configured to emit light of at least two different wavelengths, and the light sensor 122 may be configured to detect light at the at least two different wavelengths. As one example, the light emitter 120 may emit and the light sensor 122 may detect light in the visible range and light in the near-infrared or infrared range. Specifically, the light emitter 120 may emit and the light sensor 122 may detect light at 660 nm and at 910 nm. Such an embodiment may be used, for example, to ensure optimal penetration of blood vessel V and the surrounding tissue T under *in vivo* conditions.

Light of additional wavelengths may also be emitted and sensed. For example, if the method of detection is found to be sensitive to varying rates of blood flow in the vessel of interest, light at 810 nm (i.e., at the isobestic point) may be emitted and sensed to permit normalization of the results to limit or eliminate the effects of changes in blood flow rate.

According to some embodiments, the individual light sensor 122 is configured to generate a signal comprising a first pulsatile component and a second non-pulsatile component. It will be recognized that the first pulsatile component may be an alternating current (AC) component of the signal, while the second non-pulsatile component may be a direct current (DC) component. Where the light sensor 122 is in the form of an array, the pulsatile and non-pulsatile information may be generated for each element of the array, or at least for each element of the array that defines the at least one row of the array.

As to the pulsatile component, it will be recognized that a blood vessel may be described as having a characteristic pulsation of approximately 60 pulses (or beats) per minute. While this may vary with the patient's age and condition, the range of pulsation is typically between 60 and 100 pulses (or beats) per minute. The light sensor 122 will produce a signal (that is passed to the controller 124) with a particular AC waveform that corresponds to the movement of the blood through the vessel. In particular, the AC waveform corresponds to the light absorbed or reflected by the pulsatile blood flow within the vessel. On the other hand, the DC component corresponds principally to light absorbed, reflected and/or scattered by the superficial tissues.

The controller 124 is coupled to the light sensor 122, and includes a splitter 126 to separate the first pulsatile component from the second non-pulsatile component from a signal from the at least one light sensor. The splitter 126 may separate the first pulsatile component from the second non-pulsatile component for each element of the light sensor array 122. The controller 124 also includes an analyzer 128 to analyze the pulsatile and/or non-pulsatile component. The analyzer 128 may analyze the pulsatile and/or non-pulsatile component to determine the presence of and/or characteristic(s) of the vessel V within the region 102 proximate to the working end 104 of the surgical instrument 106 based (at least in part) on the pulsatile component.

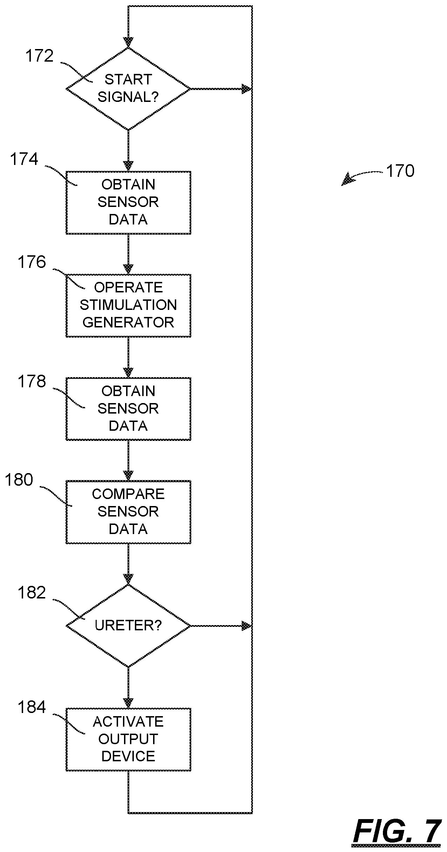

Fig. 7 illustrates a method 170 for operating the system 100 to make a determination whether tissue or an artifact, for example a ureter, is in the surgical field. According to the illustrated embodiment, where the stimulation generator 116 is controlled by the controller 124, the method 170 may be carried out by the controller 124, the controller 124 coordinating the operation of the stimulation generator 116 and the at least one sensor. As such, as to those embodiments where the controller 124 includes a processor and memory, the method 170 may be stored as a set of instructions in the memory, which set of instructions when executed by the processor causes the steps of the method 170 to be performed.

The method 170 begins at block 172, where the controller 124 waits for a start signal. According to the illustrated embodiment, the start signal is used to coordinate the stimulation generator 116 and the sensor. Such coordination is necessary according to the illustrated embodiment because the determination as to whether a ureter is present or not is dependent upon a comparison of sensor data before and after the application of stimulation to the tissue (e.g., ureter). Consequently, coordination of the stimulation generator 116 and the sensor in a chronological sense is part of the illustrated method 170, although it need not be required by every method of operation of the system 100. If no start signal is received, the method 170 remains at block 172.

Once a determination is made at block 172 that the start signal has been received, the controller 124 obtains sensor data (e.g., a first set of sensor data) from the at least one sensor, for example from the light sensor(s) 122 associated with the light emitter 120, at block 174. According to some embodiments, the at least one sensor may be operational at all times during the method 170. According to other embodiments, the at least one sensor may be operational only during the time periods before and after the operation of the stimulation generator 116 and the application of the stimulation to the tissue or artifact. In addition, it will be recognized that the activity of block 174 may include the controller 124 obtaining sensor data from more than one sensor; for example, the controller 124 may obtain sensor data from the light-based sensor including the light emitters 120 and light sensor 122 and from an impedance-based sensor. The controller 124 may obtain the sensor data from these sensors at the same time, or may obtain data from one of the sensors and then the other.

After the controller 124 has obtained the sensor data at block 174, the controller 124 operates the stimulation generator 116 to apply a stimulation to the tissue-of-interest, e.g., the ureter. The controller 124 may do this by opening and closing a switch, for example, to apply an electrical current across the electrodes 118. The amount of time that elapses between the activity at block 174 and the activity at block 176 may be selected to limit the likelihood that the sensor data will be influenced by the operation of the stimulation generator 116. After the controller 124 has operated the stimulation generator 116 to apply the stimulation at block 176, the method 170 continues to block 178.

At block 178, the controller 124 obtains sensor data (e.g., a second set of sensor data) from the at least one sensor after the stimulation has been applied. The comments made relative to the controller 124 obtaining the sensor data at block 174 apply equally to the activity at block 178. The amount of time that elapses between the activity at block 176 and the activity at block 178 again may be selected to limit the likelihood that the sensor data will be influenced by the operation of the stimulation generator 116.

At block 180, the sensor data obtained before the application of the stimulation to the tissue is compared with the sensor data obtained after the application of the stimulation to the tissue. Based on the comparison, a determination is made at block 182 whether a ureter is present within the surgical field. If a determination is made that a ureter is present, then the method continues to block 184, and the controller activates an associated output device, such as is referenced below, to provide an indication (e.g., visual, audible, or tactile) to the operator (e.g., surgeon) to alert the operator to the presence of the ureter in the surgical field. If the determination is made that the ureter is not present, then the method returns to block 172, where the controller again waits for a start signal to repeat the steps of the method 170.

The sensor data that is obtained and compared in the method 170 may vary according to the sensor included in the system 100. Similarly, while reference has been made herein to other applications describing systems and methods of operation for light-based sensors, which systems and methods may permit a determination to be made as to the presence and characteristics (e.g., diameter) of tissues and/or artifacts, it is not necessary that the sensors be operated according to the entirety of the methods described therein to be useful herein. For example, while the light-based sensor (emitters 120/sensor 122) may provide sensor data that may permit the controller 124 to determine the size and location of a vessel in the surgical field for the period before and after the stimulation is applied, it is not necessary that the controller 124 operate to determine the size and location of the vessel to make the determination that a ureter is present. Variation of the sensor data, or in specific parts of the sensor data, may permit a meaningful comparison and determination to be made, even if the size and location of the ureter is not determined. According to other embodiments, the presence, size and location of the ureter may be determined as part of the method 170.

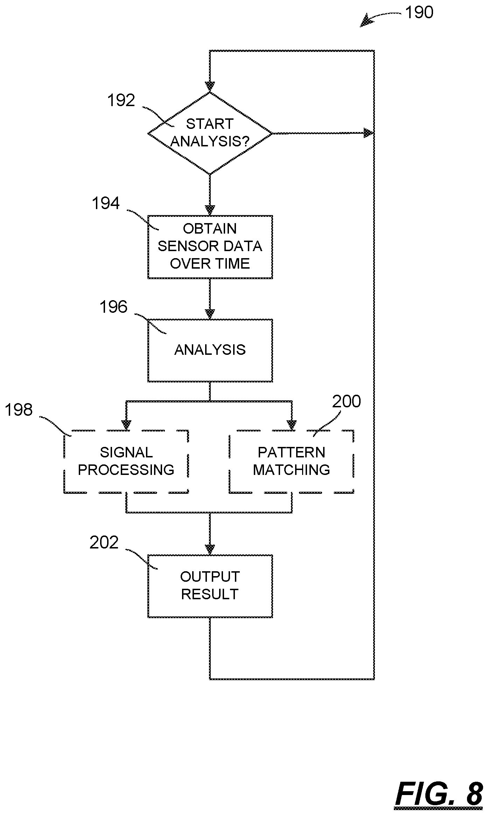

As to light-based sensors, Fig. 8 illustrates a method 190 that may be included as part of or integrated into the method 170 illustrated in Fig. 7, or may be used in the alternative to the method 170 of Fig. 7. The method 190 may begin at block 192, where the method 190 determines if an input has been received to start the analysis. For example, the input to the method 190 may be the operation of the stimulation generator 118, or may be a signal that is received by the stimulation generator 118 to initiate the stimulation. Until the input (or trigger) is received, the method 190 may loop at block 192.

Once the input is received at block 192, the method 190 continues at block 194, where sensor data is obtained or collected over time. For example, the data from the sensors 122 may be obtained over a period of time, such as several seconds. Thus, according to certain embodiments, the sensor data may be obtained for five seconds, while according to other embodiments, the sensor data may be obtained for three seconds. Further alternatives are also possible. According some embodiments, the beginning of the time period may extend before the stimulation generator is operated, and thus the data obtained may include at least one set before the stimulation generator is operated and at least one set after the stimulation generator is operated. According other embodiments, the time period may extend from shortly after the stimulation generator is operated to some point later in time.

It is presently believed that collecting the sensor data over time will permit the detection of the ureter and the separation of the response of the ureter from the response of other tissues proximate to the ureter. Some of these tissues will have a very weak response to the stimulation, remaining almost static. Other tissues, such as the bowel, which has smooth muscle tissue like the ureter, may exhibit a response over time that is different from that of the ureter, and so it is presently believed that the difference in response may be used to separate the ureter from the bowel, for example.

Once the sensor data has been obtained at block 194, the sensor data is analyzed at block 196. According to certain embodiments, the derivative of the time response of the sensor data may be used to analyze the data. As one such example, where a two-dimensional sensor array of light sensor (e.g., a camera) is used, a frame-by-frame comparison may be used to identify the changes over time, and then time derivative plots used for analysis. According to other embodiments, time-frequency analysis methods may be used, such as wavelet, short-time Fourier transform (STFT), and Wigner-Ville analysis methods. For a time-frequency analysis, it is presently believed that a high sampling rate (e.g., on the order of 100 Hz) may provide suitable results, but other sampling rates may be used instead. According to further embodiments, the time-based analysis methods may be used in conjunction with time-frequency analysis methods.

In any event, once the analysis has been performed on the sensor data, the method 190 continues to block 198 or block 200. As illustrated in Fig. 8, either one of the actions of block 198 or block 200 may follow the analysis of block 196. Consequently, the method 190 may proceed from block 196 to block 198, or from block 196 to block 200. An embodiment may even include both the actions of block 198 and of block 200, the results of block 198 being used as a confirmation of the results of block 200, or vice versa.

At block 198, a signal processing method may be applied to the results of the sensor data analysis occurring at block 196. For example, the result of the analysis at block 196 may be compared against a threshold to determine if a ureter is present or not. Alternatively, mean squared error (MSE) processing may be performed on a tensor (matrix) including the time data for each sensor along the sensor array, with a two-dimensional tensor being used for a row of sensors (i.e. a one-dimensional array) and a three-dimensional tensor being used for a two-dimensional array of sensors. According to the latter case, a three-dimensional tensor may be limited to embodiments where the two-dimensional sensor array is not more than a few sensors (or pixels) in one of the two dimensions of the sensor array.

At block 200, pattern matching may be applied to the results of sensor data analysis occurring at block 196. This pattern matching may be combined with machine learning, such that the controller 124/analyzer 128 may learn to recognize the patterns of sensor data associated with a ureter, as opposed to a bowel, for example.

In either event, the method 190 continues to block 202 after blocks 198, 200. At block 202, an output may be provided, which output may be associated with the presence of a ureter or with the absence of a ureter. This output may be used in the method 170, for example at block 182, to make the determination as to the presence of the ureter, which determination may then lead to the activation of an output device at block 184. According to certain embodiments, the actions of blocks 182, 184 of method 170 may included in the action of block 202, such that the output provided at block 202 is a signal to activate an output device if a ureter is present.

Having discussed the system 100 in general terms and method 170, the details of the system 100 are now discussed, starting with the stimulation generator 116.

As mentioned above, the stimulation generator 116 may be separate from the surgical instrument 106, or may be integrated into the surgical instrument 106. For example, the stimulation generator 116 may be disposed and housed in the same physical housing as the remainder of the controller 124, such as the splitter 126 and analyzer 128. As such, the stimulation generator 116 may be connected to the electrodes 118 via wires or leads along the shaft 108. Placement of the generator 116 in the housing of the surgical instrument 106 would limit or eliminate the need for such wires or leads. Alternative, the stimulation generator 116 may be wholly separate from the surgical instrument 106 and controller 124, such that the generator 116 may be housed instead in an instrument or tool that is used along side the surgical instrument 106, for example. According to the illustrated embodiment, the generator 116 is housed with the controller 124, and may be controlled by the controller 124 as explained in greater detail below, which placement with the controller 124 may also permit the generator 116 to be placed remotely such that, like the controller 124, the generator 116 may be reused and may not need to be sterilized to the same extent as the remainder of the system 100 (e.g., the surgical instrument 106).

Fig. 2 illustrates an embodiment of an electrical stimulation generator 116. The generator 116 is operated to provide a pulsed electrical current across the electrodes 118. The generator 116 may be configured to provide, for example, a pulsed electrical current with the following characteristics: a pulse width of 25 to 300 ms, a frequency of 1 to 5 Hz, a pulse amplitude of 60 mV to 500V, and a pulse intensity of 5 to 200 mA. Such a pulsed electrical current is believed to be particularly suited to stimulate the smooth muscle of the ureter.

The electrical stimulation generator 116 includes a switch or a transistor 140, which switch or transistor 140 may be coupled to a controller; in the illustrated embodiment, the switch/transistor 140 is coupled to the controller 124. The switch 140 is connected between a resistor 142 and a capacitor 144, which capacitor may have a fixed value capacitance. The junction of the switch 140 and the capacitor 144 is connected to first and second resistors 146, 148, which are respectively connected to a first transformer 150 and a second transformer 152. The transformers 150, 152 may be step-up transformers with a fixed ratio.

The controller 124 operates the switch/transistor 140 to open and close to provide the desired frequency and pulse width. The resistors 142, 146, 148 may be selected to provide the desired pulse amplitude and current intensity. In fact, the resistors 142, 146, 148 may be in the form of potentiometers, to permit the pulse amplitude and current intensity to be changed or varied.

Turning next to the at least one sensor, It will be recognized that a light-based sensor using transmittance or reflectance was described above in general terms above. The transmittance-based approach will now be described in greater detail with reference to Figs. 3 and 4.

A transmittance-based embodiment is illustrated in Figs. 3 and 4, wherein the light emitter 120 may include one or more elements, as referenced above. According to an embodiment schematically illustrated in Fig. 3, the light sensor 122 may include a first light emitter 120-1, a second light emitter 120-2, and a third light emitter 120-3. All of the light emitters may be configured to emit light at a particular wavelength (e.g., 660 nm), or certain emitters may emit light at different wavelengths than other emitters. Each light emitter may be a light emitting diode, for example.

As to those embodiments wherein the light emitter 120 is in the form of an array including one or more light emitting diodes, as is illustrated in Fig. 3, the diodes may be arranged in the form of a one-dimensional, two-dimensional or three-dimensional array. An example of a one-dimensional array may include disposing the diodes along a line in a single plane, while an example of a two-dimensional array may include disposing the diodes in a plurality of rows and columns in a single plane. Further example of a two-dimensional array may include disposing the diodes along a line on or in a curved surface. A three-dimensional array may include diodes disposed in more than one plane, such as in a plurality of rows and columns on or in a curved surface.

The light sensor 122 also may include one or more elements. Again, according to the embodiment illustrated in Fig. 2, the light sensor 122 may include a first light sensor 122-1, a second light sensor 122-2, an n-th light sensor 122-n, and so on. As was the case with the light emitters 120-1, 120-2, 120-3, the light sensors 122-1, 122-2, 122-3, 122-n may be arranged in an array, and the discussion about the arrays above applied with equal force here.

In fact, where the array of light sensors 122 includes a row of light sensors (such as in Fig. 3), the array 122 may be referred to in the alternative as a linear array. The individual light sensors of the array 122 may be disposed adjacent each other, or the light sensors may be spaced from each other. It may even be possible for the individual light sensors that define a row of light sensors to be separated from each other by light sensors that define a different row or column of the array. According to a particular embodiment, however, the array may comprise a charge coupled device (CCD), and in particular linear CCD imaging device comprising a plurality of pixels. As a further alternative, a CMOS sensor array may be used.

While the emitter 120 and the sensor 122 are described as disposed at the working end 104 of the surgical instrument 106, it will be recognized that not all of the components that define the emitter 120 and the sensor 122 need be disposed at the working end of the instrument 106. That is, the emitter 120 may comprise a light emitting diode, and that component may be disposed at the working end 104. Alternatively, the emitter 120 may include a length of optical fiber and a light source, the source disposed remotely from the working end 104 and the fiber having a first end optically coupled to the source and a second end disposed at the working end 104. According to the present disclosure, such an emitter 120 would still be described as disposed at the working end 104 because the light is emitted in the direction of the tissue at the working end 104 of the instrument 106. A similar arrangement may be described for the sensor 122 wherein an optical fiber has a first end disposed facing the tissue and a second end optically coupled to other components that collectively define the sensor 122.

The system 100 may include hardware and software in addition to the emitter 120, sensor 122, and controller 124. For example, where more than one emitter 120 is used, a drive controller may be provided to control the switching of the individual emitter elements. In a similar fashion, a multiplexer may be provided where more than one sensor 122 is included, which multiplexer may be coupled to the sensors 122 and to an amplifier. Further, the controller 124 may include filters and analog-to-digital conversion as may be required.

According to certain embodiments, the splitter 126 and the analyzer 128 may be defined by one or more electrical circuit components. According to other embodiments, one or more processors (or simply, the processor) may be programmed to perform the actions of the splitter 126 and the analyzer 128. According to still further embodiments, the splitter 126 and the analyzer 128 may be defined in part by electrical circuit components and in part by a processor programmed to perform the actions of the splitter 126 and the analyzer 128.

For example, the splitter 126 may include or be defined by the processor programmed to separate the first pulsatile component from the second non-pulsatile component. Further, the analyzer 128 may include or be defined by the processor programmed to determine the presence of (or to quantify the size of, for example) the vessel V within the region 102 proximate to the working end 104 of the surgical instrument 106 based on the pulsatile and/or non-pulsatile component. The instructions by which the processor is programmed may be stored on a memory associated with the processor, which memory may include one or more tangible non-transitory computer readable memories, having computer executable instructions stored thereon, which when executed by the processor, may cause the one or more processors to carry out one or more actions.

As to the operation of such a transmittance-based system to determine tissue and/or artifact (e.g., a vessel, such as a blood vessel or a ureter) characteristics, which characteristics may include position and dimension (e.g., length, width, diameter, etc.) by way of example and not by way of limitation, US Pub. Nos. 2015/0066000 and 2017/0181701 and PCT Pub. Nos. WO 2016/134327 and WO 2016/134330. As to associated structure and operation that may address issues related to the operation of such systems, PCT Pub. Nos. WO/2017/062720 and WO 2018/044722.

Fig. 4 illustrates an embodiment of the working end 104 including both the electrodes 118 and the components of the sensor. As illustrated, each of the jaws 130, 132 may include one of the electrodes 118. The electrodes 118 may be have a central opening to permit light to pass from the light emitter 120 in the first jaw 130 to the light sensor 122 in the second jaw 132. To limit the interaction between the electrodes 120 with the remainder of the jaws 130, 132 (and the emitters 120 and sensors 122), an isolator or insulator 160 is disposed between the electrodes and the remainder of the jaw 130, 132.

As mentioned above, other sensors may be used in place of the light-based sensors described above, or may be used in conjunction with the sensors, to provide additional confirmation of the movement of the stimulated tissue or artifact, for example.

One embodiment for use with a jawed surgical instrument involves measuring a change of impedance between the jaws before and after stimulation, and then associating the change of impedance with movement in the tissue between the jaws. Such a sensor may be integrated into the stimulation generator of Fig. 2, for example, by including a voltage divider and an analog-to-digital converter. Other alternatives may rely on changes in electrical potential, or changes in resistance.

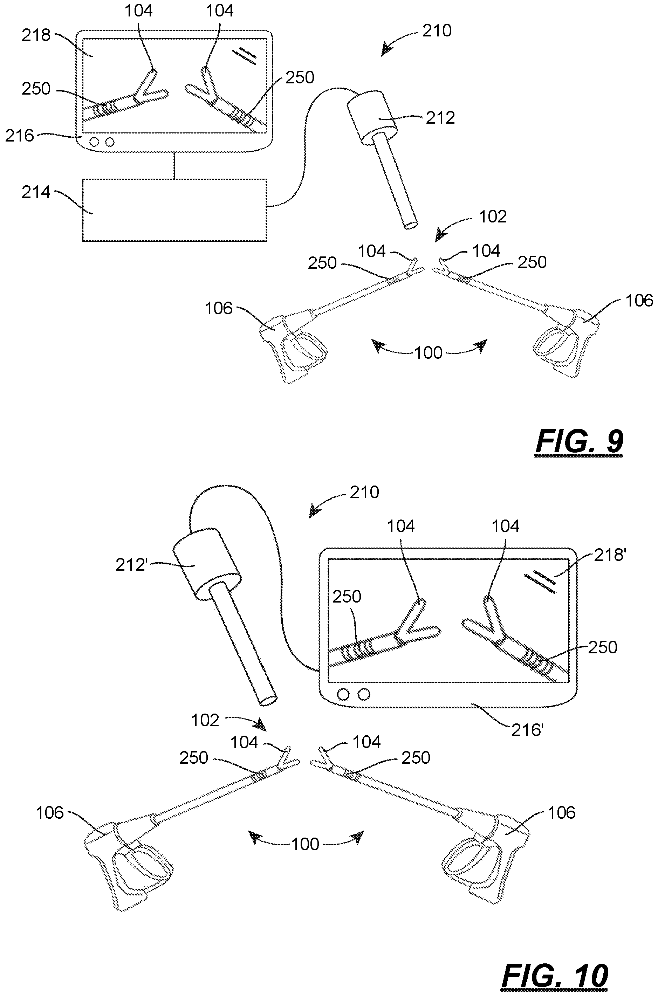

Fig. 9 and 10 illustrate an embodiment of the surgical system 100 in combination with embodiments of a video system 210, such as may be used conventionally during minimally invasive surgery or laparoscopic surgery, for example. The video system 210 includes a video camera or other image capture device 212, a video or other associated processor 214, and a display 216 having a viewing screen 218.

As illustrated, the video camera 212 is directed at the region 102 proximate the working ends 104 of two surgical instruments 106. As is also illustrated, both of the surgical instruments 106 are part of an embodiment of a surgical system 100, such as illustrated in Fig. 1 and discussed above. In this case, the instruments 106 each include a visual indicator or user interface 250, which indicator or interface 250 may include a series of bands 252, 254 that include light emitting elements 252 separated by solid bands 254 of shaft 108. It will be recognized, however, that according to other embodiments only one of the instruments 106 may include a visual indicator 250. The other elements of the surgical system 100 are omitted for ease of illustration, although it will be noted that elements of the system 100, such as the generator 116, the splitter 126 and the analyzer 128, may be housed in the same physical housing as the video processor 214, for example.

In the embodiment of Fig. 9, the signal from the video camera 212 is passed to the display 216 via the video processor 214, so that the surgeon or other member of the surgical team may view the region 102 as well as the working ends 104 of the surgical instruments 106, which are typically inside the patient. Because of the proximity of the visual indicators 250 to the working ends 104, and thus the region 102, the visual indicators 250 are also visible on the display screen 108. As mentioned previously, this advantageously permits the surgeon to receive visual cues or alarms via the visual indicators 250 via the same display 216 and on the same display screen 218 as the region 102 and the working ends 104. This, in turn, limits the need of the surgeon to look elsewhere for the information conveyed via the visual indicators 250.

Fig. 10 illustrates another embodiment of a video system 210 that can be used in conjunction with an embodiment of the surgical system 100. According to this embodiment, the video processor 214 is not disposed in a housing separate from the video camera 212', but is disposed in the same housing as the video camera 212'. According to a further embodiment, the video processor 214 may be disposed instead in the same housing as the remainder of the display 216' as the display screen 218'. Otherwise, the discussion above relative to the embodiment of the video system 210 illustrated in Fig. 9 applies equally to the embodiment of the video system 210 illustrated in Fig. 10.

While the user interface 250 advantageously permits the surgeon or surgical team to view an output from the controller 124, it is possible to include other output devices with the user interface 250, as illustrated in Fig. 1. For example, an alert may be displayed on a video display or monitor 300 being used for the surgery (e.g., the display 216, 216' in Fig. 9 and 10), or may cause an image on the monitor to change color or to flash, change size or otherwise change appearance. The auxiliary output may also be in the form of or include a speaker 302 that provides an auditory alarm. The auxiliary output also may be in the form of or may incorporate a safety lockout associated with the surgical instrument 106 that interrupts use of the instrument 106. For example, the lockout could prevent ligation or cauterization where the surgical instrument 106 is a thermal ligature device. As a still further example, the auxiliary output also may be in the form of a haptic feedback system, such as a vibrator 304, which may be attached to or formed integral with a handle or handpiece of the surgical instrument 106 to provide a tactile indication or alarm. In addition to the light emitting elements disposed at the working end 104 of the surgical instrument 108, one or more light emitting elements may be disposed at the proximal end 110 of the shaft 108, such as disposed on or attached to the grip or handle 112, to provide a visual indication or alarm. Various combinations of these particular forms of the auxiliary output may also be used.

As mentioned above, the surgical system 100 may also include the surgical instrument 106 with the working end 104, to which the user interface 250 and the sensor (and in preferred embodiments, the light emitter 120 and the light sensor 122) are attached (in the alternative, removably/reversibly or permanently/irreversibly). The user interface 250 and sensor may instead be formed integrally (i.e., as one piece) with the surgical instrument 106. As also stated, it is possible that the user interface 250 and sensor be attached to a separate instrument or tool that is used in conjunction with a surgical instrument or tool 106.

As noted above, the surgical instrument 106 may be a thermal ligature device in one embodiment. In another embodiment, the surgical instrument 106 may simply be a grasper or grasping forceps having opposing jaws. According to still further embodiments, the surgical instrument may be other surgical instruments such as irrigators, surgical staplers, clip appliers, and robotic surgical systems, for example. According to still other embodiments, the surgical instrument may have no other function that to carry the user interface and sensor and to place them within a surgical field. The illustration of a single embodiment is not intended to preclude the use of the system 100 with other surgical instruments or tools 106.

In conclusion, although the preceding text sets forth a detailed description of different embodiments of the invention, it should be understood that the legal scope of the invention is defined by the words of the claims set forth at the end of this patent. The detailed description is to be construed as exemplary only and does not describe every possible embodiment of the invention since describing every possible embodiment would be impractical, if not impossible. Numerous alternative embodiments could be implemented, using either current technology or technology developed after the filing date of this patent, which would still fall within the scope of the claims defining the invention.

It should also be understood that, unless a term is expressly defined in this patent using the sentence "As used herein, the term '____' is hereby defined to mean..." or a similar sentence, there is no intent to limit the meaning of that term, either expressly or by implication, beyond its plain or ordinary meaning, and such term should not be interpreted to be limited in scope based on any statement made in any section of this patent (other than the language of the claims). To the extent that any term recited in the claims at the end of this patent is referred to in this patent in a manner consistent with a single meaning, that is done for sake of clarity only so as to not confuse the reader, and it is not intended that such claim term be limited, by implication or otherwise, to that single meaning.

## Claims

1. A surgical system (100) comprising:
an electrical stimulation generator (116);
at least one sensor (122) disposed at a working end (104) of a surgical instrument (106); and
a controller (124) coupled to the at least one sensor (120, 122),
the controller (124) being configured to:
obtain data from the at least one sensor (122) over time in response to a stimulation applied by the electrical stimulation generator (116),
analyze the data obtained from the at least one sensor (120, 122) over time to generate sensor data analysis results;
apply a signal processing method and/or a pattern matching to the sensor data analysis results; and
indicate if a tissue (T) or artifact is present within a region (102) proximate to the working end (104) of the surgical instrument (106) based on the signal processing method or pattern matching,
**characterized in that**
the at least one sensor comprises at least one light emitter (120) and at least one light sensor (122), the at least one light emitter (120) and the at least one light sensor (122) disposed at the working end (104) of the surgical instrument (106); and
the controller (124) comprises a splitter (126) to separate a pulsatile component from a non-pulsatile component from a signal from the at least one light sensor (122) and an analyzer (128) to analyze the pulsatile and/or non-pulsatile component to generate the sensor data analysis results and apply the signal processing method and/or pattern matching to the sensor data analysis results.

2. The surgical system (100) according to claim 1, wherein the controller (124) is configured to analyze the data obtained from the at least one sensor (120,122) using time-frequency analysis methods comprising at least one of wavelet, short-time Fourier transform, and Wigner-Ville analysis methods.

3. The surgical system (100) according to claim 1 or claim 2, wherein the signal processing method comprises a comparison of the sensor data analysis results against a threshold.

4. The surgical system (100) according to claim 1 or claim 2, wherein the at least one sensor (120, 122) comprises a sensor array, and the signal processing method comprises a mean squared error processing performed on a tensor including time data for each sensor (120, 122) along the sensor array.

5. The surgical system (100) according to claim 4, wherein a two dimensional tensor is used for a one dimensional sensor array, and a three dimensional tensor is used for a two dimensional sensor array.

6. The surgical system (100) according to claim 1, wherein the electrical stimulation generator (116) is electronically connected or coupled to at least one or more surface electrodes (118), at least one or more inserted electrodes, or at least one or more intraoperative probes or needles.

7. The surgical system (100) according to claim 1 or claim 6, where the at least one sensor further comprises a sensor configured to measure the impedance between electrodes (118) of the electrical stimulation generator (116) before and after stimulation.

8. The surgical system (100) according to any one of claims 1 to 7,
wherein the at least one light sensor (122) is disposed opposite and facing the at least one light emitter (120).

9. The surgical system (100) according to claim 8, wherein the surgical instrument (106) comprises first and second jaws (130, 132), the at least one light sensor (122) disposed on the first jaw (132) and the at least one light emitter (120) disposed on the second jaw (130).

10. The surgical system (100) according to any one of claims 1 to 7,
wherein the at least one light emitter (120) and the at least one light sensor (122) face in a common direction and with fixed or variable spacing therebetween.

11. The surgical system (100) according to any one of claims 1 to 10, further comprising an associated output device (216, 218; 216', 218'; 300, 302, 304), the controller (124) is coupled to the output device (216; 218; 216', 218'; 300, 302, 304) and configured to activate the output device to alert an operator to the presence of the tissue (T) or the artifact in the region (102) proximate to the working end (104) of the surgical instrument (106).

## Patentansprüche

1. Chirurgisches System (100), das Folgendes umfasst:
einen elektrischen Stimulationsgenerator (116);
mindestens einen Sensor (122), der an einem Arbeitsende (104) eines chirurgischen Instruments (106) angeordnet ist; und
ein Steuergerät (124), das mit dem mindestens einen Sensor (120, 122) gekoppelt ist,
wobei das Steuergerät (124) zu Folgendem konfiguriert ist:
Erhalten von Daten von dem mindestens einen Sensor (122) im Laufe der Zeit als Reaktion auf eine durch den elektrischen Stimulationsgenerator (116) angelegte Stimulation,
Analysieren der von dem mindestens einen Sensor (120, 122) im Zeitverlauf erhaltenen Daten, um Sensordatenanalyseergebnisse zu erzeugen;
Anwenden eines Signalverarbeitungsverfahrens und/oder einer Musteranpassung an die Sensordatenanalyseergebnissen; und
Angeben, ob ein Gewebe (T) oder Artefakt in einem Bereich (102) in der Nähe des Arbeitsendes (104) des chirurgischen Instruments (106) vorhanden ist, basierend auf dem Signalverarbeitungsverfahren oder der Musteranpassung,
**dadurch gekennzeichnet, dass**
der mindestens eine Sensor mindestens einen Lichtemitter (120) und mindestens einen Lichtsensor (122) umfasst, wobei der mindestens eine Lichtemitter (120) und der mindestens eine Lichtsensor (122) an dem Arbeitsende (104) des chirurgischen Instruments (106) angeordnet sind; und
das Steuergerät (124) einen Splitter (126), um eine pulsierende Komponente von einer nicht-pulsierenden Komponente aus einem Signal von dem mindestens einen Lichtsensor (122) zu trennen, und einen Analysator (128) umfasst, um die pulsierende und/oder nicht-pulsierende Komponente zu analysieren, um die Sensordatenanalyseergebnisse zu erzeugen und das Signalverarbeitungsverfahren und/oder die Musteranpassung an die Sensordatenanalyseergebnisse anzuwenden.

2. Chirurgisches System (100) nach Anspruch 1, wobei das Steuergerät (124) so konfiguriert ist, dass es die von dem mindestens einen Sensor (120,122) erhaltenen Daten unter Verwendung von Zeit-Frequenz-Analyseverfahren analysiert, die mindestens eines unter Wavelet-, Kurzzeit-Fourier-Transformations- und Wigner-Ville-Analyseverfahren umfassen.

3. Chirurgisches System (100) nach Anspruch 1 oder Anspruch 2, wobei das Signalverarbeitungsverfahren einen Vergleich der Sensordatenanalyseergebnisse gegenüber einem Schwellenwert umfasst.

4. Chirurgisches System (100) nach Anspruch 1 oder Anspruch 2, wobei der mindestens eine Sensor (120, 122) ein Sensorarray umfasst und das Signalverarbeitungsverfahren eine Verarbeitung des mittleren quadratischen Fehlers umfasst, die an einem Tensor durchgeführt wird, der Zeitdaten für jeden Sensor (120, 122) entlang der Sensoranordnung beinhaltet.

5. Chirurgisches System (100) nach Anspruch 4, wobei ein zweidimensionaler Tensor für ein eindimensionales Sensorarray verwendet wird und ein dreidimensionaler Tensor für ein zweidimensionales Sensorarray verwendet wird.

6. Chirurgisches System (100) nach Anspruch 1, wobei der elektrische Stimulationsgenerator (116) mit mindestens einer oder mehreren Oberflächenelektroden (118), mindestens einer oder mehreren eingeführten Elektroden oder mindestens einer oder mehreren intraoperativen Sonden oder Nadeln elektronisch verbunden oder gekoppelt ist.

7. Chirurgisches System (100) nach Anspruch 1 oder Anspruch 6, wobei der mindestens eine Sensor ferner einen Sensor umfasst, der so konfiguriert ist, dass er die Impedanz zwischen Elektroden (118) des elektrischen Stimulationsgenerators (116) vor und nach der Stimulation misst.

8. Chirurgisches System (100) nach einem der Ansprüche 1 bis 7, wobei der mindestens eine Lichtsensor (122) dem mindestens einen Lichtemitter (120) gegenüberliegend und zugewandt angeordnet ist.

9. Chirurgisches System (100) nach Anspruch 8, wobei das chirurgische Instrument (106) eine erste und eine zweite Klemmbacke (130, 132) umfasst, wobei der mindestens eine Lichtsensor (122) an der ersten Klemmbacke (132) und der mindestens eine Lichtemitter (120) an der zweiten Klemmbacke (130) angeordnet ist.

10. Chirurgisches System (100) nach einem der Ansprüche 1 bis 7, wobei der mindestens eine Lichtemitter (120) und der mindestens eine Lichtsensor (122) in eine gemeinsame Richtung und mit festem oder variablem Abstand dazwischen zugewandt sind.

11. Chirurgisches System (100) nach einem der Ansprüche 1 bis 10, ferner umfassend eine zugehörige Ausgabevorrichtung (216, 218; 216', 218'; 300, 302, 304), wobei das Steuergerät (124) mit der Ausgabevorrichtung (216; 218; 216', 218'; 300, 302, 304) gekoppelt und so konfiguriert ist, dass es die Ausgabevorrichtung aktiviert, um einen Bediener auf das Vorhandensein des Gewebes (T) oder des Artefakts in dem Bereich (102) in der Nähe des Arbeitsendes (104) des chirurgischen Instruments (106) aufmerksam zu machen.

## Revendications

1. Système chirurgical (100) comprenant :
un générateur de stimulation électrique (116) ;
au moins un capteur (122) disposé au niveau d'une extrémité de travail (104) d'un instrument chirurgical (106) ; et
un dispositif de commande (124) couplé à l'au moins un capteur (120, 122),
le dispositif de commande(124) étant configuré pour :
obtenir des données de l'au moins un capteur (122) au fil du temps en réponse à une stimulation appliquée par le générateur de stimulation électrique (116),
analyser les données obtenues à partir de l'au moins un capteur (120, 122) au fil du temps pour générer des résultats d'analyse de données de capteur ;
appliquer un procédé de traitement de signal et/ou une mise en correspondance de modèle aux résultats d'analyse de données de capteur ; et
indiquer si un tissu (T) ou un artefact est présent dans une région (102) proche de l'extrémité de travail (104) de l'instrument chirurgical (106) sur la base du procédé de traitement de signal ou de la mise en correspondance de modèle,
**caractérisé en ce que**
l'au moins un capteur comprend au moins un émetteur de lumière (120) et au moins un capteur de lumière (122), l'au moins un émetteur de lumière (120) et l'au moins un capteur de lumière (122) étant disposés au niveau de l'extrémité de travail (104) de l'instrument chirurgical (106) ; et
le dispositif de commande (124) comprend un séparateur (126) pour séparer une composante pulsatile d'une composante non pulsatile d'un signal provenant de l'au moins un capteur de lumière (122) et un analyseur (128) pour analyser la composante pulsatile et/ou non pulsatile afin de générer les résultats d'analyse de données de capteur et d'appliquer le procédé de traitement de signal et/ou la mise en correspondance de modèle aux résultats d'analyse de données de capteur.

2. Système chirurgical (100) selon la revendication 1, dans lequel le dispositif de commande (124) est configuré pour analyser les données obtenues à partir d'au moins un capteur (120, 122) à l'aide de procédés d'analyse temps-fréquence comprenant au moins l'un des procédés d'ondelettes, de transformée de Fourier à court terme et d'analyse de Wigner-Ville.

3. Système chirurgical (100) selon la revendication 1 ou la revendication 2, dans lequel le procédé de traitement de signal comprend une comparaison des résultats d'analyse de données de capteur par rapport à un seuil.

4. Système chirurgical (100) selon la revendication 1 ou la revendication 2, dans lequel l'au moins un capteur (120, 122) comprend un réseau de capteurs, et le procédé de traitement de signal comprend un traitement d'erreur quadratique moyenne effectué sur un tenseur comprenant des données temporelles pour chaque capteur (120, 122) le long du réseau de capteurs.

5. Système chirurgical (100) selon la revendication 4, dans lequel un tenseur bidimensionnel est utilisé pour un réseau de capteurs unidimensionnels, et un tenseur tridimensionnel est utilisé pour un réseau de capteurs bidimensionnels.

6. Système chirurgical (100) selon la revendication 1, dans lequel le générateur de stimulation électrique (116) est électroniquement connecté ou couplé à au moins une ou plusieurs électrodes de surface (118), au moins une ou plusieurs électrodes insérées, ou au moins une ou plusieurs sondes ou aiguilles peropératoires.

7. Système chirurgical (100) selon la revendication 1 ou la revendication 6, où l'au moins un capteur comprend en outre un capteur configuré pour mesurer l'impédance entre les électrodes (118) du générateur de stimulation électrique (116) avant et après la stimulation.

8. Système chirurgical (100) selon l'une quelconque des revendications 1 à 7, dans lequel l'au moins un capteur de lumière (122) est disposé en face de l'au moins un émetteur de lumière (120).

9. Système chirurgical (100) selon la revendication 8, dans lequel l'instrument chirurgical (106) comprend une première et une seconde mâchoire (130, 132), l'au moins un capteur de lumière (122) étant disposé sur la première mâchoire (132) et l'au moins un émetteur de lumière (120) étant disposé sur la seconde mâchoire (130).

10. Système chirurgical (100) selon l'une quelconque des revendications 1 à 7, dans lequel l'au moins un émetteur de lumière (120) et l'au moins un capteur de lumière (122) sont orientés dans une direction commune et avec un espacement fixe ou variable entre eux.

11. Système chirurgical (100) selon l'une quelconque des revendications 1 à 10 comprend en outre un dispositif de sortie associé (216, 218 ; 216', 218' ; 300, 302, 304), le dispositif de commande (124) étant couplé au dispositif de sortie (216 ; 218 ; 216', 218' ; 300, 302, 304) et configuré pour activer le dispositif de sortie afin d'alerter un opérateur de la présence du tissu (T) ou de l'artefact dans la région (102) à proximité de l'extrémité de travail (104) de l'instrument chirurgical (106).
